# EUROPEAN PATENT APPLICATION

(11) **EP 4 160 208 A1**
(43) Date of publication of application: **05.04.2023**
(21) Application number: 21812465.9
(22) Date of filing: 18.02.2021
(51) Int. Cl.: G01N 33/493

(54) **URINE SEDIMENT STAINING LIQUID**

(30) Priority: 26.05.2020 JP 2020091318
(71) Applicant: TOYOBO CO., LTD., Osaka-shi Osaka 5300001 (JP)
(72) Inventor: OKUDA, Yohei, Osaka-shi, Osaka 530-8230 (JP); NISHIMURA, Kengo, Tsuruga-shi, Fukui 914-8550 (JP)
(74) Representative: Vossius & Partner Patentanwälte Rechtsanwälte mbB
(86) International application number: PCT/JP2021/006070
(87) International publication number: WO 2021/240914

(57) **Abstract**

An object of the present invention is to provide a method for efficiently staining a target component in a urine sediment examination by inhibiting staining of contaminants from appearing in a urine test sample. The present invention provides a supravital staining solution for urine sediment examination that contains a triarylmethane dye having an anionic functional group. In one embodiment, the triarylmethane dye is preferably a water-soluble dye. In a particularly preferable embodiment, the triarylmethane dye is used in combination with a cytoplasm-staining component. The supravital staining solution of the present invention is also excellent for staining rare elements, such as epithelial cells and casts.

## Description

### Technical Field

The present invention relates to a supravital staining solution for use in urine sediment examination, and a method using the staining solution. In particular, the present invention relates to a method for inhibiting staining of contaminants that may be contained in urine; a detection enhancement method capable of increasing the detection rate of rare elements contained in urine; and the like.

### Background Art

Urine sediment examination is important as a morphological test that is non-invasive, places less burden on the subject, and can be performed frequently. Urine sediment examination has been conventionally performed by manually preparing a slide and conducting visual assessment under microscopic examination by laboratory technicians. However, this manual operation not only imposes a heavy burden on laboratory technicians in preparation of specimens for use in microscopic examination, but also has a problem such that the judgment tends to vary depending on, for example, the laboratory technician's skill. Accordingly, as an aid to obtain uniform judgment results as well as reduce the burden on laboratory technicians, a specimen analyzer that automatically analyzes the elements of a specimen in urine sediment examination has been proposed. Specimen analyzers include flow cytometry analyzers in which a specimen and a reagent are mixed in a specimen container and the resulting suspension is passed through a flow cell to measure laser scattered light and fluorescence, and analyzers comprising an image-capturing device with which images of a specimen are captured. For example, Japanese Patent No. 3,924,870 (Patent Literature (PTL) 1) discloses preparing a test sample for detection by adding a reagent to a specimen and discloses an analyzer for capturing images of the test specimen. Further, Non-patent Literature (NPL) 1 discloses various methods for staining urine sediment.

### Citation List

### Patent Literature

PTL 1: Japanese Patent No. 3,924,870

### Non-patent Literature

NPL 1: Medical Examination, Vol. 66, No. J-STAGE-1 Special Feature: Urine Sediments, 2017, pp. 18 to 50

### Summary of Invention

### Technical Problem

In conventional urine sediment examination, not only the cell to be measured but also some contaminants that may be present in the urine are stained, which may create background noise and make it difficult to detect the target cell. In particular, when urine sediment examination is performed by using a formed elements analyzer, it was found that if contaminants that appear in the background are stained, an image may be captured in such a manner that the contaminants overlap with the component to be measured, which makes classification difficult or leads to measurement of the component based on misclassification, thus resulting in difficulty in accurately analyzing formed elements with an automated analyzer. Further in conventional urine sediment examination, rare elements, such as epithelial cells and casts, may be difficult to stain while contaminants are stained; therefore, the detection of these rare elements may be insufficient in some cases.

The present invention was made in view of the above prior art. An object of the present invention is to provide a method for inhibiting staining of contaminants in urine sediment examination to thereby suppress background noise, thus enabling efficient detection of the target cell to be measured. A further object of the present invention is to provide a method for enhancing the detection of rare elements, such as epithelial cells and casts, in a urine test sample, thus enabling highly sensitive urine sediment examination.

### Solution to Problem

As a result of intensive studies to achieve the above objects, the present inventors found that when a specific component is used in a supravital staining solution to be used in urine sediment examination, the target component can be efficiently stained without staining contaminants that appear in the background in a urine test sample. The inventors further found that more highly sensitive measurement of rare elements in urine sediment examination can be made by optimizing the mixing ratio of the supravital staining solution to urine test sample. The present invention has been accomplished based on these findings. Specifically, the present invention includes the following.
[Item 1] A supravital staining solution for urine sediment examination, comprising a triarylmethane dye having an anionic functional group.
[Item 2] The supravital staining solution for urine sediment examination according to Item 1, wherein the triarylmethane dye having an anionic functional group is a water-soluble dye.
[Item 3] The supravital staining solution for urine sediment examination according to Item 1 or 2, wherein the triarylmethane dye having an anionic functional group has a molecular weight of 700 to 900.
[Item 4] The supravital staining solution for urine sediment examination according to any one of Items 1 to 3, wherein the triarylmethane dye having an anionic functional group is at least one member selected from the group consisting of Methyl Blue, Patent Blue V, Patent Blue A, Neptune Green SBX, Acilan Brilliant Blue R, Acid First Violet BG, Brilliant Acid Blue B, Brilliant Blue R, Brilliant Blue FCF, Brilliant Blue G, Brilliant Discharge Blue G, Acid Blue, Acid Blue 13, Acid Blue 15, Acid Blue 100, Acid Blue 103, Acid Blue 104, and Acid Blue 269.
[Item 5] The supravital staining solution for urine sediment examination according to any one of Items 1 to 4, comprising Methyl Blue or Brilliant Blue G as the triarylmethane dye having an anionic functional group.
[Item 6] The supravital staining solution for urine sediment examination according to any one of Items 1 to 5, comprising Methyl Blue as the triarylmethane dye having an anionic functional group.
[Item 7] The supravital staining solution for urine sediment examination according to any one of Items 1 to 6, further comprising a cytoplasm-staining component.
[Item 8] The supravital staining solution for urine sediment examination according to Item 7, wherein the cytoplasm-staining component is at least one member selected from the group consisting of Eosin Yellowish, Eosin Bluish, Janus Green, a triphenyltetrazolium chloride solution, Neural Red, Safranin, a Pyronine B solution, Sudan III, Sudan Black, Oleyl Red, Lactofuchsin, Methyl Green, Pyronine, potassium iodide, Brilliant Cresyl Blue, Brilliant Green, Indigo Carmine, New Methylene Blue, neutral red, and Light Green.
[Item 9] The supravital staining solution for urine sediment examination according to Item 7 or 8, comprising Eosin Yellowish or Eosin Bluish as the cytoplasm-staining component.
[Item 10] The supravital staining solution for urine sediment examination according to any one of Items 7 to 9, comprising Eosin Yellowish as the cytoplasm-staining component.
[Item 11] The supravital staining solution for urine sediment examination according to any one of Items 7 to 10, comprising Methyl Blue or Brilliant Blue G as the triarylmethane dye having an anionic functional group and comprising Eosin Yellowish or Eosin Bluish as the cytoplasm-staining component.
[Item 12] The supravital staining solution for urine sediment examination according to any one of Items 7 to 11, comprising Methyl Blue as the triarylmethane dye having an anionic functional group and comprising Eosin Yellowish as the cytoplasm-staining component.
[Item 13] The supravital staining solution for urine sediment examination according to any one of Items 7 to 12, wherein the molar ratio of the cytoplasm-staining component to the triarylmethane dye having an anionic functional group is in the range of 0.5 to 8.
[Item 14] The supravital staining solution for urine sediment examination according to any one of Items 7 to 13, which is used so as to mix the supravital staining solution and a urine specimen at a ratio in the range of 1:3 to 1:9.
[Item 15] The supravital staining solution for urine sediment examination according to any one of Items 7 to 14, wherein the molar ratio of the cytoplasm-staining component to the triarylmethane dye having an anionic functional group is in the range of 0.5 to 8, and the supravital staining solution is used so as to mix the supravital staining solution and a urine specimen at a ratio in the range of 1:3 to 1:9.
[Item 16] The supravital staining solution for urine sediment examination according to any one of Items 1 to 15, further comprising a chelating agent.
[Item 17] The supravital staining solution for urine sediment examination according to any one of Items 1 to 16, which is substantially free of organic solvents.
[Item 18] The supravital staining solution for urine sediment examination according to any one of Items 1 to 17, which has a pH of 6 to 7.
[Item 19] The supravital staining solution for urine sediment examination according to any one of Items 1 to 18, which inhibits staining of contaminants in urine sediment examination.
[Item 20] The supravital staining solution for urine sediment examination according to any one of Items 1 to 19, wherein the urine sediment examination is a urine sediment examination using an image-processing urine formed elements analyzer.
[Item 21] A urine sediment examination method comprising staining a urine specimen with the supravital staining solution of any one of Items 1 to 20.
[Item 22] The urine sediment examination method according to Item 21, comprising mixing the supravital staining solution and the urine specimen at a ratio in the range of 1:3 to 1:9 to stain the urine specimen.
[Item 23] The urine sediment examination method according to Item 21 or 22, comprising capturing an image of a stained urine specimen with a urine formed elements analyzer.
[Item 24] A method for inhibiting staining of contaminants in urine sediment examination, comprising staining a urine specimen with the supravital staining solution of any one of Items 1 to 20.
[Item 25] The method for inhibiting staining of contaminants in urine sediment examination according to Item 24, wherein the supravital staining solution and the urine specimen are mixed at a ratio in the range of 1:3 to 1:9 to stain the urine specimen.
[Item 26] The method for inhibiting staining of contaminants in urine sediment examination according to Item 24 or 25, comprising capturing an image of a stained urine specimen with a urine formed elements analyzer.
[Item 27] A method for enhancing the detection of a rare element in urine sediment examination, comprising staining a urine specimen with the supravital staining solution of any one of Items 1 to 20.
[Item 28] The method for enhancing the detection of a rare element according to Item 27, wherein the rare element is at least one member selected from the group consisting of epithelial cells and casts.
[Item 29] The method for enhancing the detection of a rare element according to Item 27 or 28, wherein the supravital staining solution and the urine specimen are mixed at a ratio in the range of 1:3 to 1:9 to stain the urine specimen.
[Item 30] The method for enhancing the detection of a rare element according to any one of Items 27 to 29, comprising capturing an image of a stained urine specimen with a urine formed elements analyzer.

### Advantageous Effects of Invention

The present invention can inhibit staining of contaminants in urine sediment examination. In another aspect, the present invention enables high-sensitivity detection of a rare element, such as epithelial cells and casts, in urine sediment examination. Accordingly, highly accurate analysis can be accomplished, for example, by analyzing formed elements in a urine test sample using an automatic analyzer.

### Brief Description of Drawings

Fig. 1 is an image taken in a urine sediment examination using staining solution 1.
Fig. 2 is an image taken in a urine sediment examination using staining solution S.
Fig. 3 is an image taken in a urine sediment examination using staining solution SM.
Fig. 4 is an image taken in a urine sediment examination using staining solution PB.
Fig. 5 is an image taken in a urine sediment examination using staining solution H.
Fig. 6 is a binarized image of the image taken in a urine sediment examination using staining solution 1.
Fig. 7 is a binarized image of the image taken in a urine sediment examination using staining solution S.
Fig. 8 is a binarized image of the image taken in a urine sediment examination using staining solution SM.
Fig. 9 is a binarized image of the image taken in a urine sediment examination using staining solution PB.
Fig. 10 is a binarized image of the image taken in a urine sediment examination using staining solution H.

### Description of Embodiments

Embodiments of the present invention are described in detail below. However, the present invention is not limited thereto. It should be understood that the terms used herein have the meanings normally used in this field, unless otherwise noted.

All of the non-patent literature and patent literature described herein are incorporated herein by reference. The term "to" used herein means "... or more and ... or less." For example, "X to Y" in the present specification means X or more and Y or less. The term "and/or" in the present specification means one or the other, or both. It should be understood that throughout the present specification, the singular forms of expression also include the concept of the plural forms thereof, unless otherwise stated.

### 1. Supravital Staining Solution for Urine Sediment Examination

In one embodiment, the present invention provides a supravital staining solution for urine sediment examination, comprising a triarylmethane dye having an anionic functional group.

Urine sediment examination is an examination that observes, classifies, and measures the types and amounts of formed elements in a urine specimen, such as erythrocytes and leukocytes, to aid, for example, in the diagnosis of kidney and urinary system disorders. Traditionally, laboratory technicians have manually added and mixed a staining solution with a subject's urine specimen to prepare a prepared slide, and observed the specimen under a microscope. In order to reduce the workload of laboratory technicians and obtain uniform examination results, for example, automated urine formed elements analyzers have also been widely used in recent years. Examples of known urine sediment examinations using such a formed elements analyzer include a urine sediment examination method using an image-processing system that directly automates elements image analysis under a microscope; and a flow cytometry urine sediment examination method wherein a suspension of a urine specimen and a staining solution is passed through a flow cell to measure, for example, laser-scattered light or fluorescence. The supravital staining solution of the present invention can be used in any urine sediment examination. For example, the supravital staining solution can be used in a manual urine sediment examination or in a urine sediment examination using a formed elements analyzer. From the viewpoint of enabling more accurate determination by inhibiting staining of contaminants and also enabling detection of rare elements in mechanical discrimination without using visual inspection, the present invention is preferably used in urine sediment examination using a urine formed elements analyzer. In particular, the present invention is preferably used in urine sediment examination using an image-processing urine formed elements analyzer.

Various methods for staining biological elements are known. Among these, methods for staining cells by administering a dye into cells in vivo are called "intravital staining," whereas methods for staining cells in a living state after removing the cells from the living body are called "supravital staining." The present invention is used as a staining solution for supravital staining.

One feature of the supravital staining solution of the present invention is that the supravital staining solution of the present invention contains a triarylmethane dye having an anionic functional group. Triarylmethane dye is a generic term for dyes that have as the basic skeleton a triarylmethane structure having three benzene rings bound to one carbon. A feature of the present invention is using, from among triarylmethane dyes, a triarylmethane dye that has an anionic functional group in the structure. Examples of anionic functional groups include, but are not limited to, a sulfonic acid group, a carboxyl group, a phosphoric acid group, and a sulfate group. Preferably, a triarylmethane dye having a sulfonic acid group as an anionic functional group is used because a higher effect of the present invention can be achieved. The triarylmethane dye used in the present invention may have one or more (e.g., two or three) anionic functional groups in the structure. The triarylmethane dye used in the present invention may further contain a cationic functional group in addition to the anionic functional group described above. The present invention has been made based on the unexpected finding that when a triarylmethane dye having such an anionic functional group is used in a supravital staining solution for urine sediment examination, cell nuclei of urine formed elements can be specifically stained while staining of contaminants in the urine is inhibited.

In one embodiment, the triarylmethane dye having an anionic functional group used in the present invention is preferably a dye that is soluble in water. Being soluble in water means that the solubility in water at 20°C is, for example, 1 g/L or more, preferably 5 g/L or more, preferably 10 g/L or more, preferably 50 g/L or more, preferably 100 g/L or more, preferably 200 g/L or more, and more preferably 250 g/L or more (for example, 300 g/L or more). The upper limit of the solubility is not particularly limited; however, it may be, for example, 1000 g/L or less.

The molecular weight of the triarylmethane dye having an anionic functional group to be used in the present invention is not particularly limited as long as the effect of the invention is provided. The molecular weight is, for example, preferably about 500 to 1100, more preferably about 600 to 1000, and even more preferably about 700 to 900. By using a triarylmethane dye with such a molecular weight, high effects can be stably provided.

In one embodiment, the triarylmethane dye having an anionic functional group used in the present invention is preferably a dye that is stable around the pH of human urine (around neutral). From this viewpoint, the triarylmethane dye having an anionic functional group to be used in the present invention is preferably stable around the pH range of 6 to 8, more preferably stable around the pH range of 6 to 7, and particularly stable around the pH range of 6 to 6.5. The triarylmethane dye having an anionic functional group to be used in the present invention preferably has coloring in the pH range described above.

In another aspect, the triarylmethane dye having an anionic functional group to be used in the present invention preferably has low or no carcinogenicity from the viewpoint of ease of handling. In view of concerns about bacterial degeneration, the triarylmethane dye having an anionic functional group preferably has low or no bactericidal properties. Further, when used in combination with a cytoplasm-staining component as described below, the triarylmethane dye preferably has a color tone different from that of the cytoplasm-staining component. For example, when the cytoplasm-staining component to be used in combination is a component that has red-based coloring, such as Eosin Yellowish, a triarylmethane dye that has blue-based coloring is preferable.

Specifically, the triarylmethane dye having an anionic functional group that can be used in the present invention can be any dye known in this field. Preferable examples of the triarylmethane dye having a sulfonic acid group include Methyl Blue (also known as Acid Blue 93), Patent Blue V (also known as Acid Blue 1), Patent Blue A (also known as Acid Blue 7), Neptune Green SBX (also known as Acid Blue 11), Acilan Brilliant Blue R (Acid Blue 24), Acid First Violet BG (Acid Blue 34), Acid Brilliant Acid Blue B (also known as Acid Blue 38), Brilliant Blue R (also known as Acid Blue 83), Brilliant Blue FCF (also known as Acid Blue 9), Brilliant Blue G (also known as Acid Blue 90), Brilliant Discharge Blue G (also known as Acid Blue 91), Acid Blue, Acid Blue 13, Acid Blue 15, Acid Blue 100, Acid Blue 103, Acid Blue 104, and Acid Blue 269. The triarylmethane dye having an anionic functional group in the present invention can be preferably Methyl Blue or Brilliant Blue G, and more preferably Methyl Blue. Furthermore, in the present invention, such triarylmethane dyes having an anionic functional group can be used singly or in a combination of two or more.

Methyl Blue (CAS No. 28983-56-4) is a known compound that is represented by a molecular formula of C₃₇H₂₇N₃Na₂O₉S₃ and that is known to be highly soluble in water (solubility in water: 300 g/L (20°C), molecular weight: 799.8). Methyl Blue is known as a dye that stains connective tissues, such as collagen fibers, and collagen blue around the neutral pH. Methyl Blue is known to be neither carcinogenic nor bactericidal. The Methyl Blue to be used may be chemically synthesized, or a commercially available product. Mixtures, such as Aniline Blue, which is a mixture of Methyl Blue and Water Blue; and Lactophenol Cotton Blue, which is a mixture formed by dissolving Methyl Blue in phenol, glycerol, or a butyric acid solution, can also be preferably used as Methyl Blue in the present invention.

Brilliant Blue G (CAS No. 6104-58-1) is a known compound that is represented by a molecular formula of C₄₇H₄₈N₂[N⁺]NaO₆[O⁻]S₂ and known to be soluble in water, and that has a blue color (solubility in water: 50 g/L, molecular weight: 854.0). The Brilliant Blue G to be used may be chemically synthesized, or can be a commercially available product.

The supravital staining solution according to one embodiment of the present invention may contain only a triarylmethane dye having an anionic functional group as a staining component, or may further contain one or more other staining components. From the viewpoint of facilitating the detection of various components with higher sensitivity, the supravital staining solution of the present invention preferably contains multiple dyes. For example, the supravital staining solution of the present invention can contain a triarylmethane dye having an anionic functional group and at least one member selected from the group consisting of cell nucleus-staining components (staining components that stain at least the nucleus) and cytoplasm-staining components (staining components that stain at least cytoplasm and the like). In particular, the supravital staining solution of the present invention is preferably a supravital staining solution containing a triarylmethane dye having an anionic functional group and a cytoplasm-staining component.

The cell nucleus-staining component can be any staining component that stains at least the nucleus. Examples include, but are not limited to, orcein acetate, carmine acetate, Methylene Blue, Alcian Blue, Crystal Violet, Methyl Violet, hematoxylin, 2,7-diaminofluorene, potassium ferrocyanide, and Azure II.

The cytoplasm-staining component may be any staining component that stains at least cytoplasm and the like. Examples include, but are not limited to, Eosin Yellowish, Eosin Bluish, Janus Green, triphenyltetrazolium chloride solution, Neural Red, Safranin, Pyronine B solution, Sudan III, Sudan Black, Oleyl Red, Lactofuchsin, Methyl Green, Pyronine, potassium iodide, Brilliant Cresyl Blue, Brilliant Green, Indigo Carmine, New Methylene Blue, neutral red, and Light Green. From the viewpoint of more securely and easily obtaining the effects of the present invention, the cytoplasm-staining component is preferably an Eosin dye, such as Eosin Yellowish and Eosin Bluish, and more preferably Eosin Yellowish.

When the supravital staining solution of the present invention contains a combination of a cell nucleus-staining component and/or a cytoplasm-staining component, one cell nucleus-staining component and/or one cytoplasm-staining component may be used, or two or more nucleus-staining components and/or two or more cytoplasm-staining components may be used in combination.

In one embodiment, preferably, the supravital staining solution for urine sediment examination of the present invention, for example, contains Methyl Blue or Brilliant Blue G as the triarylmethane dye having an anionic functional group, and contains Eosin Yellowish or Eosin Bluish as a cytoplasm-staining component; however, this is not limitative. In a more preferred embodiment, the supravital staining solution for urine sediment examination, for example, contains Methyl Blue as the triarylmethane dye having an anionic functional group and contains Eosin Yellowish as the cytoplasm-staining component, although not limitative.

Examples of reagents generally known to aid in identifying formed elements in a test sample using a mixture of staining components such as those described above etc. include the Sternheimer-Malbin staining method (the SM staining method); the Sternheimer staining method (the S staining method, the NS staining method, or a variation of the Sternheimer staining method); the Prescott-Brodie staining method; the Behre-Muhlberg staining method (the BM staining method); the Sudan III staining method; the Lugol staining method; the hemosiderin staining method; the Papanicolaou staining method; the 4-chloro-1-naphthol method; the Field's staining method; the Quaglino-Flemans method; the Kaplow method; the Sato-Sekiya method; the Berlin Blue method; the Giemsa staining method; the Wright staining method; the Pappenheim staining method; the Congo Red staining method; the Methyl Green-Pyronine staining method; the Alcian Blue staining method; the Shorr staining method; the Feulgen staining method; the Oil Red O staining method; the Brecker method; the Heinz body staining method; the Neural Red-Janus Green supravital staining method; and the Brilliant Cresyl Blue staining method (*Rinsho-Kensa Atorasu 1, Nyo-Chinsa* (Atlas of Clinical Laboratory Examination, Vol. 1, Urinary Sediment); *Rinsho-Kensa Gijutsu Zensho 3, Ketsueki-Kensa* (Complete Book of Techniques of Clinical Laboratory Examination, Vol. 3, Blood Test); *Rinsho-Kensa-Ho Teiyou* (Outline of Clinical Laboratory Examination Methods); *Senshoku-Ho no Subete,* MEDICAL TECHNOLOGY Bessatsu (All About Staining Methods, Additional Volume of Medical Technology)).

The present inventors conducted extensive research on a further new staining method, and as a result, found a staining component that can stain the target without staining contaminants that appear in the background in an analyzer for capturing images of urine formed elements. Specifically, the inventors found that when a triarylmethane dye having an anionic functional group is used as a component for staining nuclei, high-sensitivity detection of target cells can be achieved while inhibiting staining of urine contaminants in urine sediment examination. As shown in the results of the Test Examples described below, this result was ascertained by confirming that when a triarylmethane dye having an anionic functional group is used in combination with, for example, Eosin Yellowish or Eosin Bluish as a cytoplasm-staining component, a more preferable effect can be obtained. In a further specific aspect, it has become clear that the supravital staining solution of the present invention can enhance the detection sensitivity of rare elements, such as epithelial cells and/or casts, which generally tend to be difficult to stain by conventional methods.

When the supravital staining solution of the present invention contains a cytoplasm-staining component, the mixing ratio of the cytoplasm-staining component to the triarylmethane dye having an anionic functional group is not particularly limited as long as the effect of the present invention is provided. For example, the mixing ratio of the cytoplasm-staining component (preferably Eosin Yellowish, Eosin Bluish, etc.) to the triarylmethane dye having an anionic functional group is preferably in the range of 0.2 to 10, more preferably in the range of 0.4 to 9, even more preferably in the range of 0.5 to 8, and still even more preferably in the range of 1 to 4, in terms of molar ratio.

The content of the triarylmethane dye having an anionic functional group in the supravital staining solution of the present invention is also not limited as long as the effect of the invention is provided. The content of the triarylmethane dye having an anionic functional group is, for example, 1.0 to 15 mmol/L, preferably 1.5 to 12.0 mmol/L, and more preferably 2.0 to 6.0 mmol/L.

When the supravital staining solution of the present invention further contains a cytoplasm-staining component, the content of the cytoplasm-staining component is also not particularly limited as long as the effect of the invention is provided. The content of the cytoplasm-staining component (preferably Eosin Yellowish, Eosin Bluish, etc.) is 0.3 to 12.0 mmol/L, preferably 1.0 to 12.0 mmol/L, and more preferably 4.0 to 12.0 mmol/L.

The supravital staining solution of the present invention preferably further contains a chelating agent to reduce the influence of amorphous salts contained in a urine specimen. In particular, when images are captured by an image-processing urine formed elements analyzer, clearer images can be captured by suppressing the influence of amorphous salts in this manner. The chelating agent to be used can be any chelating agent known in this field. Examples include ethylenediaminetetraacetic acid (EDTA), nitrilotriacetic acid (NTA), diethylenetriaminepentaacetic acid (DTPA), hydroxyethylethylenediaminetriacetic acid (HEDTA), triethylenetetraaminehexaacetic acid (TTHA), 1,3-propanediaminetetraacetic acid (PDTA), 1,3-diamidino-2-hydroxypropanetetraacetic acid (DTPA-OH), hydroxyethyliminodiacetic acid (HIDA), dihydroxyethylglycine (DHEG), glycoletherdiaminetetraacetic acid (EGTA), dicarboxymethylglutamic acid (CMGA), (S,S)-ethylenediaminesuccinic acid (EDDS), hydroxyethylidene diphosphonic acid (HEDP), nitrilotris(methylenephosphonic acid) (NTMP), phosphonobutanetricarboxylic acid (PBTC), ethylenediaminetetra(methylenephosphonic acid) (EDTMP), gluconic acid, O,O'-bis(2-aminophenyl)ethyleneglycol-N,N,N'-tetraacetic acid, tetrapotassium salt, hydrate (BAPTA), N,N-bis(2-hydroxyethyl)glycine (Bicine), trans-1,2-diaminocyclohexane-N,N,N',N'-tetraacetic acid, monohydrate (CyDTA), iminodiacetic acid (IDA), nitrilotriacetic acid (NTA), N,N,N',N'-tetrakis(2-pyridylmethyl)ethylenediamine (TPEN), and salts thereof (e.g., disodium ethylenediaminetetraacetate, trisodium hydrogen ethylenediaminetetraacetate, and hydrate salts thereof). When such a chelating agent is added, the content of the chelating agent is not particularly limited as long as the effect of the present invention is provided. The content is, for example, 1 to 50 mmol/L, preferably 5 to 30 mmol/L, and more preferably 10 to 25 mmol/L.

The supravital staining solution of the present invention may be a solution obtained by dissolving the aforementioned staining component in any solvent. The solvent to be used herein is not particularly limited as long as the effect of the present invention can be provided. The solvent can be, for example, any liquid, such as water, inorganic solvents, or organic solvents. From the viewpoint of not atrophying the cell components to be observed, the solvent is preferably water or inorganic solvents rather than organic solvents, such as ethanol, used in the SM staining method, and is particularly preferably water.

In a specific embodiment, the supravital staining solution for urine sediment examination of the present invention is preferably substantially free of organic solvents. In the present specification, being substantially free of organic solvents means that the content of organic solvents in the supravital staining solution is less than 5 volume%, preferably less than 3 volume%, and more preferably less than 1 volume%.

The supravital staining solution may further contain a buffer in order to stabilize the staining components contained in the solution and adjust the pH when a test sample and the staining solution are mixed. Examples of such buffering solutions include Tris buffers, phosphate buffers, borate buffers, and Good's buffers. Good's buffers are more preferred because the pH of Tris buffers, phosphate buffers, and borate buffers are prone to fluctuate depending on the concentration and temperature.

Good's buffers are zwitterionic buffers, most of which are prepared by using an amphoteric electrolyte amino acid, in particular, a substituted taurine or N-substituted glycine, as a buffering agent. Some Good's buffers may include aliphatic amines or non-zwitterionic buffers. Examples of such Good's buffers include MES (pKa 6.15), Bis-tris (pKa 6.46), ADA (pKa 6.80), PIPES (pKa 6.80), ACES (pKa 6.90), cholamine chloride (pKa 7.10), BES (pKa 7.15), MOPS (pKa 7.20), TES (pKa 7.50), HEPES (pKa 7.55), HEPPS (pKa 8.00), Tricine (pKa 8.15), glycinamide (pKa 8.20), Bicine (pKa 8.35), TAPS (pKa 8.40), CHES (pKa 9.50), and CAPS (pKa 10.40).

The buffer used in the present invention is preferably a Good's buffer having a buffering capacity around the neutral pH in order to increase the stability of staining components, and is more preferably a Good's buffer having a buffering capacity at pH 6 to 8.

The buffer used in the present invention is more preferably a Good's buffer having a buffer capacity at pH 6.0 to 7.0. The reason therefor is that the urine of a healthy person is generally considered to have a pH around neutral (usually around pH 6.0 to 6.5); and when a urine specimen is mixed with the staining solution, the state of formed elements in urine can be analyzed in a more intact state.

Specifically, preferable buffers include MES (pKa 6.15), Bis-tris (pKa 6.46), ADA (pKa 6.80), PIPES (pKa 6.80), and ACES (pKa 6.90).

The supravital staining solution of the present invention may further contain one or more preservatives, salts, staining component stabilizers, and the like, as long as such additives do not cause any adverse effects.

Examples of preservatives include ProClin 150, ProClin 200, ProClin 300, ProClin 950, azides, chelating agents, antibiotics, and antimicrobial agents.

Examples of antibiotics include gentamicin, kanamycin, and chloramphenicol.

Examples of antimicrobial agents include methylisothiazolinone and imidazolidinylurea.

Examples of salts include sodium chloride, potassium chloride, and aluminum chloride.

Examples of staining component stabilizers include cyclodextrins.

The test sample used in the analysis of urine sediment examination of the present invention may be a test sample derived from urine collected from a mammal, including humans (also referred to as urine specimen). For example, any test sample, such as raw urine, concentrated urine, or urine sediment after centrifugation, can be used in the present invention. Further, the supravital staining solution of the present invention can be used not only for urine specimens but also for biological body fluids, such as blood, abdominal fluid, and spinal fluid, as well as beverages, foods, and the like that people ingest.

When urine sediment examination is performed using the supravital staining solution of the present invention, the mixing ratio of the supravital staining solution of the present invention to a test sample is not particularly limited and can be, for example in the range of 1:1 to 1:10, preferably 1:3 to 1:9, and particularly preferably 1:3 to 1:6. As shown in the results of the test examples described below, mixing the supravital staining solution and the urine specimen in such a ratio can make it possible to enhance the detection of rare elements, such as epithelial cells and/or casts, in urine sediment examination.

Accordingly, when the supravital staining solution of the present invention is used in such a manner that a test sample and the supravital staining solution were mixed at a ratio descried above, the supravital staining solution can also be considered to be a supravital staining solution for urine sediment examination that is used to improve the detection of rare elements in urine sediment examination.

Examples of epithelial cells observed in urine sediment examination include, but are not limited to, renal tubular epithelial cells, urothelial cells, columnar epithelial cells, squamous epithelial cells, and glomerular epithelial cells. Examples of casts observed in urine sediment examination include vitreous casts, epithelial casts, granular casts, wax-like casts, fat casts, erythrocyte casts, leukocyte casts, vacuolar degeneration casts, salt crystal casts, macrophage casts, fibrin casts, hemosiderin casts, myoglobin casts, and Bence-Jones protein casts. By varying the mixing ratio of the supravital staining solution of the present invention to a test sample, the detection rate of such rare elements of epithelial cells and/or casts can be increased.

### 2. Urine Sediment Examination Method

The supravital staining solution of the present invention can be used in any urine sediment examination method in which supravital staining is performed. In one embodiment, the urine sediment examination method of the present invention can comprise the step of staining a urine specimen with the supravital staining solution of the present invention described above. The urine sediment examination method as referred to herein may be, for example, a urine sediment examination in which the supravital staining is performed manually by a laboratory technician, or a urine sediment examination performed using a urine formed elements analyzer with which supravital staining is automatically performed. The urine sediment examination method using a urine formed elements analyzer with the supravital staining solution may be a urine sediment examination by flow cytometry, or may be a urine sediment examination using an image-processing urine formed elements analyzer that directly automates the analysis of urine formed elements under a microscope. From the viewpoint of inhibiting staining of contaminants in urine to reduce background noise and easily capturing an image of the target cell while focusing on the target cell, and reducing the misclassification of formed elements in urine, the urine sediment examination method is preferably a urine sediment examination using an image-processing urine formed elements analyzer.

In a specific embodiment, the urine sediment examination using a urine formed elements analyzer performed in the present invention comprises the step of staining a urine specimen with the supravital staining solution of the present invention described above. This staining step can be performed by any method known in this field. For example, a urine specimen can be stained by adding and mixing the supravital staining solution and the urine specimen in a predetermined proportion in a container, such as a sample tube or a reaction tube, or placing the supravital staining solution and the urine specimen on a glass slide (e.g., a sediment plate, a microscopic plate) used for capturing an image and mixing the staining solution and the sample. Before being subjected to urine sediment examination, a urine specimen is preferably stirred and then used. In a container in which a urine specimen has been collected, the supravital staining solution of the present invention may be added and mixed. Alternatively, the supravital staining solution may be added beforehand and a urine specimen may be added thereto and mixed.

The supravital staining solution of the present invention has been found to enhance the detection of rare elements, such as epithelial cells and/or casts, when mixed with a urine specimen at a predetermined ratio. Accordingly, when there is a strong need to enhance the detection of such rare elements, the supravital staining solution and urine specimen are preferably mixed at a ratio in the range of 1:3 to 1:9, and more preferably in the range of 1:3 to 1:6, to stain the urine specimen.

Accordingly, in one embodiment, the supravital staining solution for urine sediment examination of the present invention is a supravital staining solution for urine sediment examination that is used so as to mix the supravital staining solution and a urine specimen at a ratio in the range of 1:3 to 1:9. In a particularly preferred embodiment, the supravital staining solution of the present invention is a supravital staining solution for urine sediment examination, wherein the solution contains a triarylmethane dye having an anionic functional group and a cytoplasm-staining component, the molar ratio of the cytoplasm-staining component to the triarylmethane dye having an anionic functional group is in the range of 0.5 to 8, and the supravital staining solution is used so as to mix the supravital staining solution and a urine specimen at a ratio in the range of 1:3 to 1:9.

In one embodiment, the urine sediment examination using a urine formed elements analyzer performed in the present invention comprises the step of capturing an image of a stained urine specimen using a urine formed elements analyzer. The image-capturing step can be carried out by using a urine formed elements analyzer equipped with a means for capturing an image of a stained urine specimen under a microscope. For example, the image-capturing step can be performed by using a urine formed elements analyzer comprising a stage for capturing an image of formed elements in a urine specimen stained with the supravital staining solution; a means for magnifying the image of the prepared slide of formed elements in the urine specimen; and a means for focusing on the image of the prepared slide and capturing the image; and optionally further comprising a means for processing the captured image to identify each formed element etc. It was known that in urine sediment examination using such an image-processing urine formed elements analyzer, detailed classification of epithelial cells and casts may be insufficient, although this may be different depending on the model of analyzer used (Non-patent Literature (NPL) 1). According to the present invention, enhanced detection of such epithelial cells and casts can also be achieved; therefore, the supravital staining solution of the present invention is particularly useful for urine sediment examination using an image-processing urine formed elements analyzer.

### 3. Methods for Inhibiting Staining of Contaminants in Urine Sediment Examination

As described above, according to the present invention, staining of contaminants in urine sediment examination can be highly inhibited by using the supravital staining solution containing a specific dye.

Thus, according to another aspect, the present invention provides a method for inhibiting staining of contaminants in urine sediment examination, the method comprising the step of staining a urine specimen with the supravital staining solution for urine sediment examination of the present invention. The application method, such as the type and concentration of the triarylmethane dye having an anionic functional group used in this method; the type and concentration of other optional components that can be incorporated, such as a cytoplasm-staining component; and the mixing ratio of the supravital staining solution of the present invention to a urine specimen, are the same as those described above in sections "1. Supravital Staining Solution for Urine Sediment Examination" and "2. Urine Sediment Examination Method."

### 4. Method for Enhancing the Detection of Rare elements in Urine Sediment Examination

As described above, according to the present invention, the detection of rare elements, such as epithelial cells and/or casts, whose detection in urine sediment examination is known to tend to be insufficient, can be improved by using the supravital staining solution containing a specific dye and mixing the supravital staining solution and a urine specimen in a specific ratio.

Thus, according to still another aspect, the present invention provides a method for enhancing the detection of a rare element in urine sediment examination, the method comprising the step of staining a urine specimen with the supravital staining solution for urine sediment examination of the present invention. The application method, such as the type and concentration of the triarylmethane dye having an anionic functional group used in this method; the type and concentration of other optional components that can be incorporated, such as a cytoplasm-staining component; and the mixing ratio of the supravital staining solution of the present invention to a urine specimen, are the same as those described above in sections "1. Supravital Staining Solution for Urine Sediment Examination" and "2. Urine Sediment Examination Method."

### Examples

The effects of the present invention are shown below with reference to Examples. However, the present invention is not limited to these Examples.

### Example 1

### Comparison of Staining Solutions Suitable for Formed Elements Analyzer

Using urine as a test sample and using the following staining solutions and analyzer, the staining solutions were examined for their suitability for use in a formed element analyzer. Each staining solution and sample were mixed at a ratio of 1:4, and measurement was carried out.

### Preparation of Staining Solution

A staining solution having the following formulation was prepared.

### Staining solution 1

| | |
|---|---|
| MES-NaOH | 100 mM pH 6.3 |
| Methyl Blue | 3 mmol/L |
| Eosin Yellowish | 6 mmol/L |

For comparison, Sternheimer stain (staining solution S), Sternheimer-Malbin stain (staining solution SM), and Prescott-Brodie staining method (staining solution PB), Hansel stain (staining solution H) were used.

### Measurement

Using a USCANNER (registered trademark) (E) urine formed elements analyzer (produced by Toyobo) and the above staining solutions 1, S, SM, PB, and H, a urine test sample was measured in accordance with the measurement method described in the package insert of the analyzer.

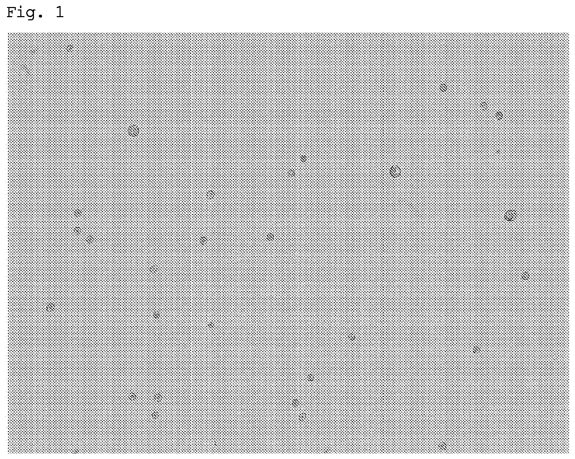

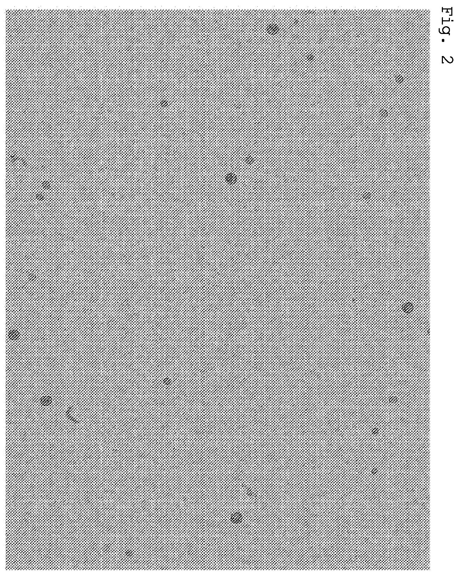

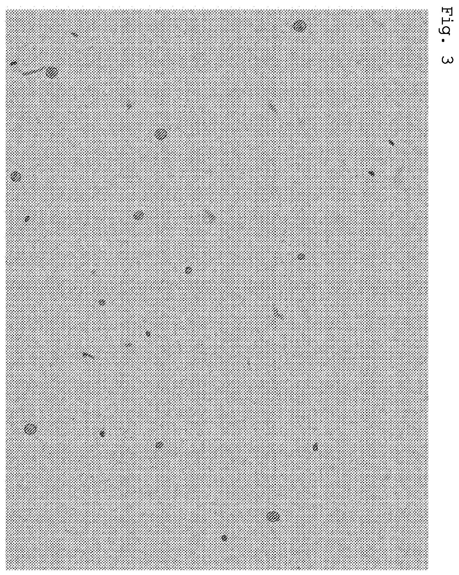

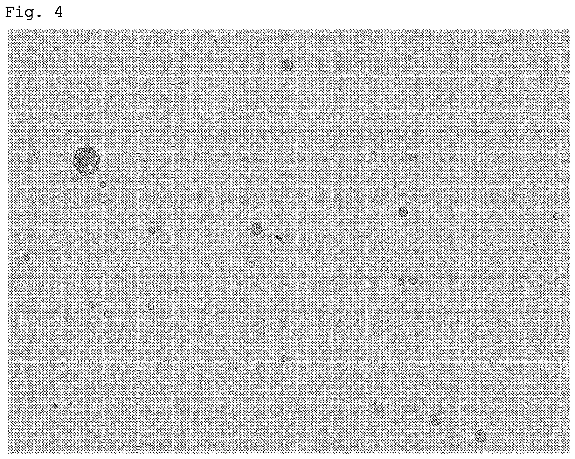

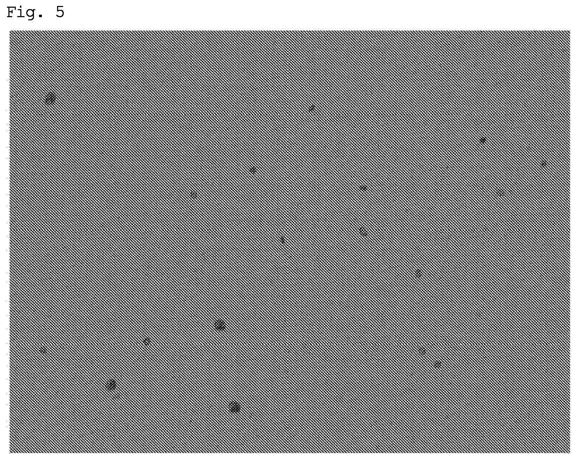

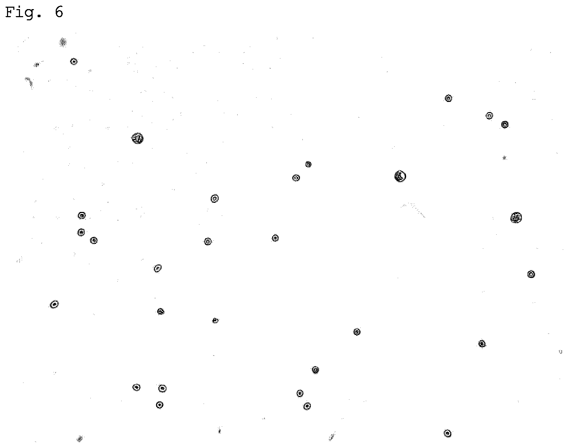

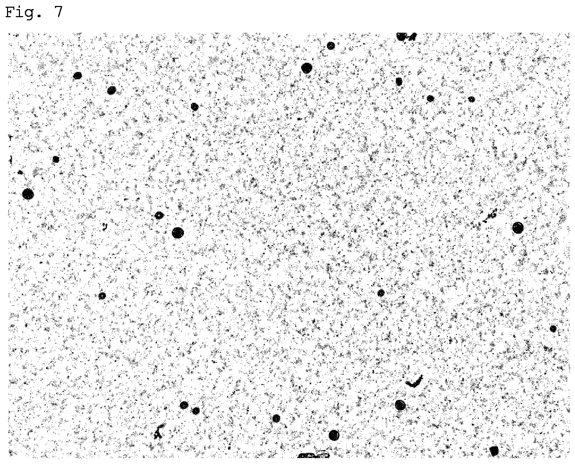

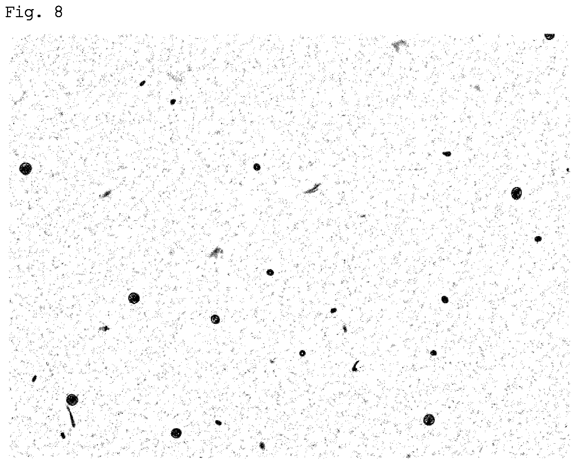

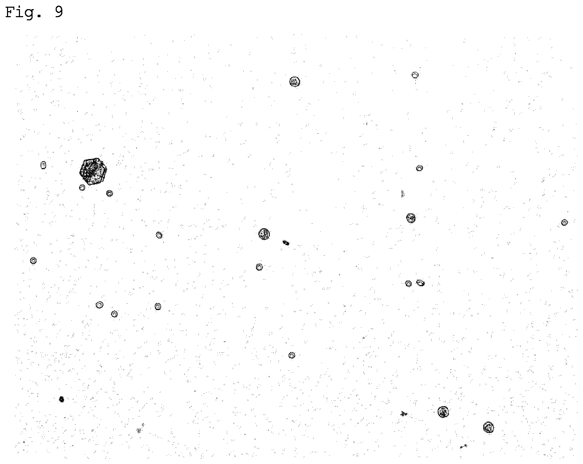

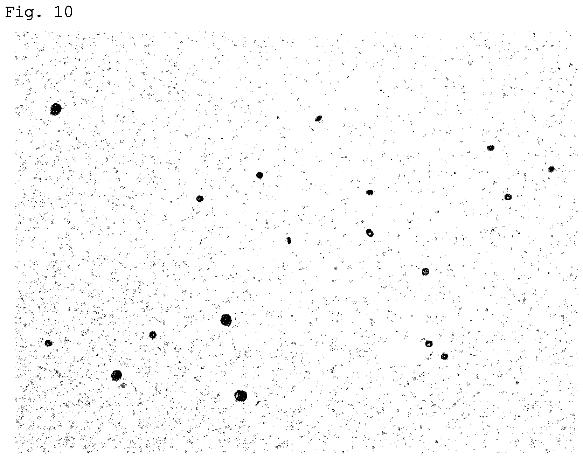

Figs. 1 to 5 show the results of capturing images of the urine test sample. In order to further clarify the degree of staining of background contaminants, Figs. 6 to 10 show binarized images obtained by using the staining solutions. As shown in the figures, the staining solutions S, SM, PB, and H stained contaminants appearing in the background, thus causing misidentification of formed elements and making accurate analysis with the analyzer difficult, whereas staining solution 1 did not stain contaminants and was confirmed to achieve more accurate analysis of formed elements. Staining solution SM refers to a staining solution prepared by mixing Crystal Violet, which is a triarylmethane dye not containing an anionic functional group, and safranin O in 95% ethanol. Staining solution H refers to a staining solution prepared by mixing Methylene Blue, which is a phenothiazine dye, and Eosin Yellowish in a phosphate-buffered physiological saline containing methanol and ethanol. The results of this Example show that it is important to contain at least a triarylmethane dye having an anionic functional group (Methyl Blue).

### Example 2

### Examination 1 of Mixing Ratio of Staining Components

The following staining solutions were prepared by changing the mixing ratio of the staining components. Measurements were performed in the same manner as in Example 1 using urine as a test sample and using a USCANNER (registered trademark) (E) analyzer. The mixing ratio of staining components in a staining solution suitable for a formed elements analyzer was examined. The staining solution and the sample were mixed at a ratio of 1:3. As a control, the same urine test sample was visually observed under a microscope.

### Preparation of Staining Solutions

### Staining solutions 2 to 8

| | |
|---|---|
| PIPES-NaOH | 100 mM pH 6.5 |
| Methyl Blue (MB) | See Table 1 below. |
| Eosin Yellowish (EY) | See Table 1 below. |

**Table 1**

| | Staining solution 2 | Staining solution 3 | Staining solution 4 | Staining solution 5 | Staining solution 6 | Staining solution 7 | Staining solution 8 |
|---|---|---|---|---|---|---|---|
| MB (mmol/L) | 12.0 | 6.0 | 3.0 | 2.0 | 1.5 | 2.0 | 1.0 |
| EY (mmol/L) | 6.0 | 6.0 | 6.0 | 8.0 | 12.0 | 0.3 | 10.0 |
| EY/MB ratio | 0.5 | 1.0 | 2.0 | 4.0 | 8.0 | 0.2 | 10.0 |

Table 2 shows the analysis results of the urine test sample. The results confirmed that among the staining solutions examined, staining solutions 2 to 6 achieved good analysis results that are equivalent to the control results obtained by microscopic observation, whereas accurate analysis of the formed elements was difficult to achieve when staining solution 7 or staining solution 8 was used. The above results show that a preferred mixing ratio of Eosin Yellowish to Methyl Blue is in the range of 0.5 to 8 in terms of molar ratio.

**Table 2**

| | Staining solution 2 | Staining solution 3 | Staining solution 4 | Staining solution 5 | Staining solution 6 | Staining solution 7 | Staining solution 8 | Microscopic observation |
|---|---|---|---|---|---|---|---|---|
| Erythrocyte (/HPF) | ≥ 100 | ≥ 100 | ≥ 100 | ≥ 100 | ≥ 100 | 30-49 | 50-99 | ≥100 |
| Leucocyte (/HPF) | 50-99 | 50-99 | 50-99 | 50-99 | 50-99 | 30-49 | 20-29 | 50-99 |
| Epithelial cells (/HPF) | <1 | <1 | <1 | <1 | <1 | <1 | <1 | <1 |
| Casts (/LPF) | <1 | <1 | <1 | <1 | <1 | <1 | <1 | <1 |
| Microbes (/HPF) | <1 | <1 | <1 | <1 | <1 | <1 | <1 | <1 |
| Crystals (/HPF) | 5-9 | 5-9 | 5-9 | 5-9 | 5-9 | <1 | <1 | 5-9 |

### Example 3

### Analysis and Examination of Rare elements

Whether rare elements can be detected was examined by changing the mixing ratio of a staining solution to a test sample. As the staining solution, staining solution 9 described below was used. Measurements were performed in the same manner as in Example 1 using urine as the sample and using a USCANNER (registered trademark) (E) analyzer. As a control, the staining solution and the test sample were mixed at a ratio of 1:4, and the same urine test sample was visually observed under a microscope.

### Preparation of Staining Solution

A staining solution comprising the following formulation was prepared.

### Staining solution 9

| | |
|---|---|
| MES-NaOH | 100 mM pH 6.0 |
| Tripotassium hydrogen ethylenediaminetetraacetate | 20 mmol/L |
| Methyl Blue | 2 mmol/L |
| Eosin Yellowish | 4 mmol/L |

Table 3 shows the analysis results of the urine test sample. The results confirmed that as compared to the results obtained by microscopic observation, more detectable elements were found as the stain:specimen ratio was increased from 1:9 to 1:3. In particular, the detection of rare elements, such as epithelial cells (renal tubular epithelium was confirmed in this example) and casts, was confirmed to be improved. Even when the ratio of the sample was increased and the ratio of the staining solution was reduced, the staining solution of the present invention was confirmed to be able to be sufficiently stain the formed elements.

**Table 3**

| | Staining solution : Sample | | | Microscopic observation |
|---|---|---|---|---|
| | 1:3 | 1:6 | 1:9 | |
| Erythrocyte (/HPF) | ≥ 100 | ≥ 100 | ≥ 100 | ≥ 100 |
| Leucocyte (/HPF) | ≥ 100 | 50-99 | 50-99 | 50-99 |
| Epithelial cells (/HPF) | 1-4 | 1-4 | <1 | <1 |
| Casts (/LPF) | 5-9 | 1-4 | <1 | <1 |
| Bacteria (/HPF) | 5-9 | 5-9 | 1-4 | <1 |
| Crystals (/HPF) | 5-9 | 5-9 | 1-4 | 1-4 |

### Example 4

### Examination 2 of Mixing Ratio of Staining Components

To confirm whether the same effect can also be achieved by using a triarylmethane dye having an anionic functional group different from the above triarylmethane dye, the following test was performed.

First, staining solutions containing staining components at different mixing ratios as shown below were prepared. In this example, Brilliant Blue G was used as a triarylmethane dye having an anionic functional group and Eosin Bluish was used as a cytoplasm-staining component. Using urine as a test sample and using a USCANNER (registered trademark) (E) analyzer, measurements were performed to evaluate the mixing ratio of staining components in a staining solution suitable for the formed elements analyzer. Each staining solution and sample were mixed at a ratio of 1:3. As a control, the same urine test sample was visually observed under a microscope.

### Preparation of Staining Solutions

### Staining solutions 10 to 16

| | |
|---|---|
| PIPES-NaOH | 100 mM pH 6.5 |
| Tripotassium hydrogen ethylenediaminetetraacetate | 20 mmol/L |
| Brilliant Blue G (BG) | See Table 4 below. |
| Eosine Bluish (EB) | See Table 4 below. |

**Table 4**

| | Staining solution 10 | Staining solution 11 | Staining solution 12 | Staining solution 13 | Staining solution 14 | Staining solution 15 | Staining solution 16 |
|---|---|---|---|---|---|---|---|
| BG (mmol/L) | 12.0 | 6.0 | 3. | 2.0 | 1.5 | 2.0 | 1.0 |
| EG (mmol/L) | 6.0 | 6.0 | 6.0 | 8.0 | 12.0 | 0.3 | 10.0 |
| EB/BG ratio | 0.5 | 1.0 | 2.0 | 4.0 | 8.0 | 0.2 | 10.0 |

Table 5 shows the analysis results of the urine test sample. The results confirmed that when a staining solution containing a triarylmethane dye having an anionic functional group, which is soluble like Methyl Blue and has a molecular weight of 700 to 900 (Brilliant Blue G), and a cytoplasm-staining component were mixed in predetermined amounts, good analysis results equivalent to the control results obtained by microscopic observation were obtained. More specifically, the results confirmed that when staining solutions 10 to 14 were used, the analysis results equivalent to the control results obtained by microscopic observation were obtained, whereas accurate analysis of formed elements was difficult to achieve by using staining solution 15 or staining solution 16. The above results show that the mixing ratio of Eosin Bluish to Brilliant Blue G is preferably in the range of 0.5 to 8 in terms of molar ratio. The results of this test example confirmed that that similar effects can also be achieved by a combination of any triarylmethane dye having an anionic functional group and any cytoplasm-staining component.

**Table 5**

| | Staining solution 10 | Staining solution 11 | Staining solution 12 | Staining solution 13 | Staining solution 14 | Staining solution 15 | Staining solution 16 | Microscopic observation |
|---|---|---|---|---|---|---|---|---|
| Erythrocyte (/HPF) | ≥ 100 | ≥ 100 | ≥ 100 | ≥ 100 | ≥ 100 | 30-49 | 50-99 | >100 |
| Leucocyte (/HPF) | 30-49 | 30-49 | 30-49 | 30-49 | 30-49 | 20-29 | 10-19 | 30-49 |
| Epithelial cells (/HPF) | <1 | <1 | <1 | <1 | <1 | <1 | <1 | <1 |
| Casts (/LPF) | <1 | <1 | <1 | <1 | <1 | <1 | <1 | <1 |
| Microbes (/HPF) | <1 | <1 | <1 | <1 | <1 | <1 | <1 | <1 |
| Crystals (/HPF) | 1-4 | 1-4 | 1-4 | 1-4 | 1-4 | <1 | <1 | 1-4 |

### Example 5: Examination of Combination of Staining Components

Further, the following staining solutions were prepared by changing the combination of staining components. Using urine as a test sample and using a USCANNER (registered trademark) (E) analyzer, measurements were performed to examine what mixing ratio of staining components in a staining solution is suitable for the formed elements analyzer. Each staining solution and sample were mixed at a ratio of 1:3. As a control, the same urine test sample was visually observed under a microscope.

### Preparation of Staining Solutions

### Staining solution 17

| | |
|---|---|
| PIPES-NaOH | 100 mM pH 6.5 |
| Tripotassium hydrogen ethylenediaminetetraacetate | 20 mmol/L |
| Methyl Blue (MB) | 3 mmol/L |
| Eosin Bluish (EB) | 6 mmol/L |

### Staining solution 18

| | |
|---|---|
| PIPES-NaOH | 100 mM pH 6.5 |
| Tripotassium hydrogen ethylenediaminetetraacetate | 20 mmol/L |
| Brilliant Blue G (BG) | 3 mmol/L |
| Eosin Yellowish (EY) | 6 mmol/L |

Table 6 shows the analysis results of the urine test sample. The results confirmed that when using a staining solution prepared by mixing a triarylmethane having an anionic functional group (Methyl Blue or Brilliant Blue G) and a cytoplasm-staining component (Eosin Bluish or Eosin Yellowish) in predetermined amounts, good analysis results equivalent to the control results obtained by microscopic observation were obtained. The above results confirmed that the effect of the present invention can be achieved by any combination of a triarylmethane having an anionic functional group and a cytoplasm-staining component.

**Table 6**

| | Staining solution 17 | Staining solution 18 | Microscopic examination |
|---|---|---|---|
| Erythrocyte (/HPF) | ≥ 100 | ≥ 100 | ≥ 100 |
| Leucocyte (/HPF) | 50-99 | 50-99 | 50-99 |
| Epithelial cells (/HPF) | <1 | <1 | <1 |
| Casts (/LPF) | <1 | <1 | <1 |
| Microbes (/HPF) | <1 | <1 | <1 |
| Crystals (/HPF) | 1-4 | 1-4 | 1-4 |

### Industrial Applicability

The present invention enables accurate measurement and more advanced analysis of rare elements in analysis by capturing images of formed elements in a test sample using a formed element analyzer.

## Claims

1. A supravital staining solution for urine sediment examination, comprising a triarylmethane dye having an anionic functional group.

2. The supravital staining solution for urine sediment examination according to claim 1, wherein the triarylmethane dye having an anionic functional group is a water-soluble dye.

3. The supravital staining solution for urine sediment examination according to claim 1 or 2, wherein the triarylmethane dye having an anionic functional group has a molecular weight of 700 to 900.

4. The supravital staining solution for urine sediment examination according to any one of claims 1 to 3, wherein the triarylmethane dye having an anionic functional group is at least one member selected from the group consisting of Methyl Blue, Patent Blue V, Patent Blue A, Neptune Green SBX, Acilan Brilliant Blue R, Acid First Violet BG, Brilliant Acid Blue B, Brilliant Blue R, Brilliant Blue FCF, Brilliant Blue G, Brilliant Discharge Blue G, Acid Blue, Acid Blue 13, Acid Blue 15, Acid Blue 100, Acid Blue 103, Acid Blue 104, and Acid Blue 269.

5. The supravital staining solution for urine sediment examination according to any one of claims 1 to 4, comprising Methyl Blue or Brilliant Blue G as the triarylmethane dye having an anionic functional group.

6. The supravital staining solution for urine sediment examination according to any one of claims 1 to 5, comprising Methyl Blue as the triarylmethane dye having an anionic functional group.

7. The supravital staining solution for urine sediment examination according to any one of claims 1 to 6, further comprising a cytoplasm-staining component.

8. The supravital staining solution for urine sediment examination according to claim 7, wherein the cytoplasm-staining component is at least one member selected from the group consisting of Eosin Yellowish, Eosin Bluish, Janus Green, a triphenyltetrazolium chloride solution, Neural Red, Safranin, a Pyronine B solution, Sudan III, Sudan Black, Oleyl Red, Lactofuchsin, Methyl Green, Pyronine, potassium iodide, Brilliant Cresyl Blue, Brilliant Green, Indigo Carmine, New Methylene Blue, neutral red, and Light Green.

9. The supravital staining solution for urine sediment examination according to claim 7 or 8, comprising Eosin Yellowish or Eosin Bluish as the cytoplasm-staining component.

10. The supravital staining solution for urine sediment examination according to any one of claims 7 to 9, comprising Eosin Yellowish as the cytoplasm-staining component.

11. The supravital staining solution for urine sediment examination according to any one of claims 7 to 10, comprising Methyl Blue or Brilliant Blue G as the triarylmethane dye having an anionic functional group and comprising Eosin Yellowish or Eosin Bluish as the cytoplasm-staining component.

12. The supravital staining solution for urine sediment examination according to any one of claims 7 to 11, comprising Methyl Blue as the triarylmethane dye having an anionic functional group and comprising Eosin Yellowish as the cytoplasm-staining component.

13. The supravital staining solution for urine sediment examination according to any one of claims 7 to 12, wherein the molar ratio of the cytoplasm-staining component to the triarylmethane dye having an anionic functional group is in the range of 0.5 to 8.

14. The supravital staining solution for urine sediment examination according to any one of claims 7 to 13, which is used so as to mix the supravital staining solution and a urine specimen at a ratio in the range of 1:3 to 1:9.

15. The supravital staining solution for urine sediment examination according to any one of claims 7 to 14, wherein the molar ratio of the cytoplasm-staining component to the triarylmethane dye having an anionic functional group is in the range of 0.5 to 8, and the supravital staining solution is used so as to mix the supravital staining solution and a urine specimen at a ratio in the range of 1:3 to 1:9.

16. The supravital staining solution for urine sediment examination according to any one of claims 1 to 15, further comprising a chelating agent.

17. The supravital staining solution for urine sediment examination according to any one of claims 1 to 16, which is substantially free of organic solvents.

18. The supravital staining solution for urine sediment examination according to any one of claims 1 to 17, which has a pH of 6 to 7.

19. The supravital staining solution for urine sediment examination according to any one of claims 1 to 18, which inhibits staining of contaminants in urine sediment examination.

20. The supravital staining solution for urine sediment examination according to any one of claims 1 to 19, wherein the urine sediment examination is a urine sediment examination using an image-processing urine formed elements analyzer.

21. A urine sediment examination method comprising staining a urine specimen with the supravital staining solution of any one of claims 1 to 20.

22. The urine sediment examination method according to claim 21, comprising mixing the supravital staining solution and the urine specimen at a ratio in the range of 1:3 to 1:9 to stain the urine specimen.

23. The urine sediment examination method according to claim 21 or 22, comprising capturing an image of a stained urine specimen with a urine formed elements analyzer.

24. A method for inhibiting staining of contaminants in urine sediment examination, comprising staining a urine specimen with the supravital staining solution of any one of claims 1 to 20.

25. The method for inhibiting staining of contaminants in urine sediment examination according to claim 24, wherein the supravital staining solution and the urine specimen are mixed at a ratio in the range of 1:3 to 1:9 to stain the urine specimen.

26. The method for inhibiting staining of contaminants in urine sediment examination according to claim 24 or 25, comprising capturing an image of a stained urine specimen with a urine formed elements analyzer.

27. A method for enhancing the detection of a rare element in urine sediment examination, comprising staining a urine specimen with the supravital staining solution of any one of claims 1 to 20.

28. The method for enhancing the detection of a rare element according to claim 27, wherein the rare element is at least one member selected from the group consisting of epithelial cells and casts.

29. The method for enhancing the detection of a rare element according to claim 27 or 28, wherein the supravital staining solution and the urine specimen are mixed at a ratio in the range of 1:3 to 1:9 to stain the urine specimen.

30. The method for enhancing the detection of a rare element according to any one of claims 27 to 29, comprising capturing an image of a stained urine specimen with a urine formed elements analyzer.
